# EUROPEAN PATENT SPECIFICATION

(11) **EP 2 080 749 B1**
(45) Date of publication and mention of the grant of the patent: **05.05.2021**
(21) Application number: 08790499.1
(22) Date of filing: 25.08.2008
(51) Int. Cl.: C07C 29/34, C07C 31/02, C07C 31/125, C10L 1/02, C07B 61/00

(54) **METHOD OF SYNTHESIZING CHEMICAL INDUSTRY RAW MATERIALS AND FUEL COMPOSITIONS**
VERFAHREN ZUR SYNTHESE EINES CHEMISCH-INDUSTRIELLEN ROHMATERIALS ODER EINER KRAFTSTOFFZUSAMMENSETZUNG
PROCÉDÉ DE SYNTHÈSE D'UNE MATIÈRE PREMIÈRE INDUSTRIELLE CHIMIQUE OU D'UNE COMPOSITION DE COMBUSTIBLE

(30) Priority: 24.08.2007 JP 2007219064
(43) Date of publication of application: 22.07.2009
(73) Proprietor: KABUSHIKI KAISHA SANGI, Tokyo 104-8440 (JP)
(72) Inventor: TSUCHIDA, Takashi, Tokyo 104-8440 (JP); SAKUMA, Shuji, Tokyo 104-8440 (JP); YOSHIOKA, Tetsuya, Tokyo 104-8440 (JP); KUBO, Jun, Tokyo 104-8440 (JP)
(74) Representative: D Young & Co LLP
(86) International application number: PCT/JP2008/002295
(87) International publication number: WO 2009/028166

(56) References cited:
- EP-A1- 1 052 234
- WO-A1-99/38822
- WO-A1-2006/059729
- CA-A1- 2 589 123
- CA-A1- 2 589 125
- JP-A- H0 418 042
- JP-A- H03 279 336
- JP-A- 2004 261 751
- CARLINI C ET AL: "Guerbet condensation of methanol with n-propanol to isobutyl alcohol over heterogeneous bifunctional catalysts based on Mg-Al mixed oxides partially substituted by different metal components", JOURNAL OF MOLECULAR CATALYSIS A: CHEMICAL, ELSEVIER, AMSTERDAM, NL, vol. 232, no. 1-2, 3 May 2005 (2005-05-03) , pages 13-20, XP027658287, ISSN: 1381-1169 [retrieved on 2005-05-03]

## Description

### Technical Field

The present invention relates to a method for synthesizing 1 or more kinds of branched-chain alcohol useful as a chemical industry raw material and fuel composition or a mixture thereof.

### Background Art

Presently, oxo process comprising synthesizing normal aldehyde by oxidation of normal paraffin and hydrogenating the obtained aldehyde is the mainstream of methods for synthesizing industrial linear alcohol. However, as the price of naphtha, raw material of normal paraffin, is escalating, the profitability is decreasing. Besides the oxo method, a method using methanol (alcohol) and synthetic gas (carbon monoxide and hydrogen) as raw materials is known. However, as carbon monoxide which is harmful is used in the method and that it is a high-pressure reaction, the plant is of a large scale and the profitability is not good. Further, Ziegler method comprising oligomerizing ethylene by trialkylaluminum, forming a long-chain aluminum alkoxide by air-oxidation, and hydrolyzing the resultant to obtain a long-chain primary alcohol is used. With that method, only alcohol having even numbers of carbon atoms having a distribution of 2 - 28 carbon atoms can be obtained. Moreover, a method for synthesizing 1-propanol from methanol and ethanol by Guerbet method has been proposed, while the yield is not good, as the reaction conditions are specific and thus not suitable for practical use. Furthermore, alcohol is also synthesized from plants such as copra oil (oleochemical), while only alcohol having 8 or 16 carbon atoms can be obtained, and for alcohol having other numbers of atoms, it is necessary to depend on naphtha.

As a method for synthesizing higher alcohol from methanol and ethanol, a method using ununiformed catalysts such as MgO can be exemplified (see nonpatent documents 1-5, patent documents 1-4), while these methods are not suitable for industrialization as they are many side reaction products, or the reaction conditions are specific. Further, as a method for synthesizing butanol from ethanol, a method using oxidative products of alkaline-earth metals as catalysts (see nonpatent document 6), a method using zeolite substituted with alkaline metal (see nonpatent document 7), a method using a mixture of metal oxidative products (see nonpatent document 8) can be exemplified. As for a method for manufacturing butadiene from ethanol, a method using a metal oxidative product or a mixture thereof (see nonpatent documents 9-11), a method using a sepiolite catalyst which is a cellular acicular clay can be exemplified. However, these methods are not industrially suitable as the catalysts are difficult to prepare, or that the reaction temperature is high.

On the other hand, a method for synthesizing butanol, butadiene, or fuel compositions by using a hydroxyapatite catalyst(see patent documents 7, 8) has been proposed, while as it is a method using only ethanol as raw material, organic compounds that can be synthesized were limited. In other words, as ethanol is a material having 2 carbon atoms, it is not suitable for synthesizing organic compounds having odd numbers of carbon atoms, and particularly, alcohol having odd numbers of carbon atoms cannot be synthesized.

US 6323383 discloses a process for the synthesis of chemical industrial feedstock and high-octane fuel, wherein calcium phosphate which is controlled in the molar Ca/P ratio and/or one which contains an activating metal (M) at a molar (Ca+M)/P ratio of 1 to 2 is used as the catalyst and ethanol is used as the feedstock.

CA 2589125 discloses a clean process for efficiently producing, from ethanol as a raw material, higher-molecular alcohols having an even number of carbon atoms, such as 1-butanol, hexanol, octanol, and decanol and a mixture of these.

[Nonpatent document 1] Ueda, W.; Kuwabara, T.; Ohshida, T.; Morikawa, Y. A Low-pressure Guerbet Reaction over Magnesium Oxide Catalyst. J. Chem. Soc., Chem. Commun., 1990, 1558-1559.
[Nonpatent document 2] Ueda, W.; Ohshida, T.; Kuwabara, T.; Morikawa, Y. Condensation of alcohol over solid-base catalyst to form higher alcohols. Catal. Letters, 1992, 12, 97-104.
[Nonpatent document 3] Olson, E. S., Sharma, R. K. and Aulich T. R. Higher-Alcohols Biorefinery Improvement of Catalyst for Ethanol Conversion. Applied Biochemistry and Biotechnology, 2004, vol. 113-116, 913-932.
[Nonpatent document 4] Burk, P. L.; Pruett, R. L. and Campo, K. S. The Rhodium-Promoted Guerbet Reaction Part 1. Higher Alcohols from Lower Alcohols. J. of Molecular Catalysis, 1985, 33, 1-14.
[Nonpatent document 5] Knothe, G. Synthesis, applications, and characterization of Guerbet compounds and their derivatives. Lipid Technology, 2002, September, 101-104.
[Nonpatent document 6] "Dimerisation of ethanol to butanol over solid-base catalysts" A.S. Ndou, N. plint, N. J. Coville, Applied catalysis A: General, 251, p. 337-345 (2003)
[Nonpatent document 7] "Bimolecular Condensation of Ethanol to 1-Butanol Catalyzed by Alkali Cation Zeolites" C. Yang, Z. Meng, J. of Catalysis, 142, p. 37-44 (1993)
   Carlini et al, 2005, Journal of Molecular Catalysis A: Chemical, 232, pages 13-20 relates to guerbet condensation of methanol with n-propanol to isobutyl alcohol over heterogeneous bifunctional catalysts based on Mg-Al mixed oxides partially substituted by different metal components.
[Nonpatent document 8] "Kinetics of a Complex Reaction System-Preparation of n-Butanol from Ethanol in One Step, V. NAGARAJAN, Indian Journal of Technology Vol. 9, October 1971, pp. 380-386
[Nonpatent document 9] "BUTADIENE FROM ETHYL ALCOHOL" B. B. CORSON, H. E. JONES, C. E. WELLING, J. A. HINCLEY, AND E. E. STAHLY, INDUSTRIAL AND ENGINEERING CHEMISTRY, Vol. 42. No.2
[Nonpatent document 10] ONE - STEP CATALYTIC CONVERSION OF ETHANOL TO BUTADIENE IN THE FIXED BED. I. SINGLE-OXIDE CATALYSIS, S. K. BHATTACHARYYA and N. D. GANGULY, J. appl. Chem. ,12, March, 1962)
[Nonpatent document 11] ONE-STEP CATALYTIC CONVERSION OF ETHANOL TO BUTADIENE IN THE FIXED BED. II. BINARY- AND TERNARY-OXIDE CATALYSIS, S. K. BHATTACHARYYA and N. D. GANGULY, J. appl. Chem., 12, March, 1962)

[Patent document 1] US 2971033
[Patent document 2] US 3972952
[Patent document 3] US 5300695
[Patent document 4] US 2050788
[Patent document 5]Japanese Laid-Open patent Application No. 57-102822
[Patent document 6] Japanese Laid-Open patent Application No. 58-59928
[Patent document 7] WO99/38822
[Patent document 8] WO2006/059729

### Disclosure of the Invention

### Object to be solved by the Invention

The object of the present invention is to provide a novel method for synthesizing 1 or more kinds of branched-chain alcohol comprising allowing methanol and alcohol having 3 or more carbon atoms to contact hydroxyapatite at normal pressure and at 200 - 600°C, wherein the hydroxyapatite is not one supporting metal catalysts or metal ion catalysts acting on alcohol. Particularly, it is to provide a method for synthesizing branched-chain alcohol in good yield.

### Means to Solve the Object

The present inventors made a study for manufacturing organic compounds to be used as a chemical industry raw material, and found out that by using hydroxyapatite or hydrotalcite as a catalyst, various organic compounds can be manufactured from 2 or more kinds of alcohol, or from 1 kind of alcohol having 3 or more carbon atoms.

Further, the present inventors made a keen study for synthesizing linear alcohols, under conditions that almost all of the alcohols synthesized by using alcohol raw material were branched-chain alcohols, and that it was estimated to be extremely difficult to synthesize linear alcohols. As a result, they found out that by allowing ethanol and linear alcohol other than ethanol to contact hydroxyapatite or hydrotalcite, a linear alcohol can be synthesized in good yield. Currently, ethanol is synthesized through the conversion of sugars obtained from sugarcanes, beets, etc., by a fermentation method. Recently, a technique for synthesizing ethanol from biomass, agricultural and forestry residues, has been established, and a striking increase in the production of ethanol can be expected in future. Further, as the production cost of ethanol is becoming comparable or less than the crude oil, it is an important object to synthesize chemical industry raw materials using ethanol as a raw material. The process described herein uses ethanol derived from plants as a raw material, and the reaction proceeds easily at normal pressure. Thus, comparing with the conventional synthesizing method using fossil or mineral resource, which emit carbon dioxide and promote global heating, as a raw material, it is an important synthesizing method for global environment.

Further, the present inventors found out that by allowing methanol and alcohol having 3 or more carbon atoms to contact hydroxyapatite or hydrotalcite, a branched-chain alcohol can be synthesized in good yield. The present invention is defined in the appended claims and relates a method for synthesizing 1 or more kinds of branched-chain alcohol comprising allowing methanol and alcohol having 3 or more carbon atoms to contact hydroxyapatite at normal pressure and at 200 - 600°C, wherein the hydroxyapatite is not one supporting metal catalysts or metal ion catalysts acting on alcohol.

Also described but not claimed herein is ("1") a method for synthesizing 1 or more kinds of organic compounds comprising allowing 2 or more kinds of alcohols to contact hydroxyapatite (except those supporting metal catalysts or metal ion catalysts acting on alcohol) ; ("2") the method according to "1", wherein at least 1 kind of alcohol is methanol or ethanol; ("3") the method according to "1" or "2", wherein a linear alcohol having 3 or more carbon atoms is synthesized by allowing ethanol and liner alcohol other than ethanol to contact hydroxyapatite; ("4") the method according to "3", wherein the linear alcohol other than ethanol is methanol, 1-propanol, 1-butanol, 1-pentanol, 1-hexanol, 1-heptanol, 1-octanol, or unsaturated alcohols thereof; ("5") the method according to "3" or "4", wherein the yield of the synthesized linear alcohol is 3C-mol % or more; ("6") the method according to ("1") or ("2"), comprising allowing methanol and alcohol having 3 or more carbon atoms to contact hydroxyapatite to synthesize branched-chain alcohol; ("7") the method according to ("6"), wherein the alcohol having 3 or more carbon atoms is a linear alcohol; ("8") the method according to "7", wherein the linear alcohol is 1-propanol, 1-butanol, 1-pentanol, 1-hexanol, 1-heptanol, 1-octanol, or unsaturated alcohols thereof.

Further, described herein is ("9") a method for synthesizing 1 or more kinds of organic compounds, comprising allowing 1 kind of alcohols having 3 or more carbon atoms to contact hydroxyapatite (except those supporting metal catalysts or metal ion catalysts acting on alcohol) ; ("10") the method according to "9", wherein the alcohol having 3 or more carbon atoms is propanol, butanol, pentanol, hexanol, heptanol, octanol, or unsaturated alcohols thereof; ("11") the method according to "1", "2", "9" or "10", wherein the synthesized organic compound is a fuel composition; ("12") the method according to any one of "1" to "11", wherein the reaction is conducted at 200 - 600°C.

Further, described herein is ("13") a method for synthesizing 1 or more kinds of organic compounds comprising allowing 2 or more kinds of alcohol to contact hydrotalcite; ("14") the method according to "13", comprising allowing ethanol and linear alcohol other than ethanol to contact hydrotalcite to synthesize a linear alcohol having 3 or more carbon atoms; ("15") the method according to "14", wherein the linear alcohol other than ethanol is methanol, 1-propanol, 1-butanol, 1-pentanol, 1-hexanol, 1-heptanol, 1-octanol, or unsaturated alcohols thereof.

### Brief Explanation of Drawings

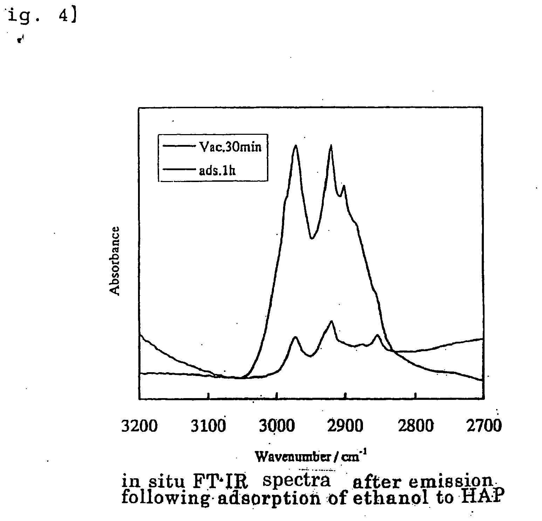

[Fig. 1]
   It is a figure showing the yield of alcohol against the reaction temperature, when ethanol and 1-propanol (ethanol : 1-propanol = 1 : 1) are used as raw material alcohol.
[Fig. 2]
   It is a figure showing the yield of alcohol against the reaction temperature, when ethanol and 1-propanol (ethanol : 1-propanol = 1 : 4) are used as raw material alcohol.
[Fig. 3]
   It is a figure showing the yield of alcohol against the reaction temperature, when ethanol and 1-propanol (ethanol : 1-propanol = 4 : 1) are used as raw material alcohol.
[Fig. 4]
   It is a figure showing the results of measurement of the inner state of the reactor by in situ FT-IR after 1 hour of exposure to ethanol/He mixed gas, followed by 30 min of emission.
[Fig. 5]
   It is a figure showing the detailed results after 30 min of emission of Fig. 4.

### Best Mode for Carrying Out the Synthesizing Method

As for a method for synthesizing an organic compound described herein (first synthesizing method), it is not particularly limited as long as it is a method comprising allowing 2 or more kinds of alcohols to contact hydroxyapatite (except those supporting metal catalysts or metal ion catalysts acting on alcohol) . Examples of organic compounds synthesized by the method for synthesizing described herein include: paraffins, olefins, dienes, trienes, alcohols, ethers, ketones, aldehydes, and esters. Specific examples include: ethane, ethylene, acetaldehyde, propylene, propanol, acetone, butene, 1,3-butadiene, 1-butanol, 3-butene-1-ol, t-crotylalcohol, c-crotylalcohol, diethylether, butyraldehyde, 2-butanone, t-crotonealdehyde, c-crotonaldehyde, 1-pentanol, 2-pentanol, 2-pentanone, butylethylether, 1-hexanol, 2-ethyl-1-butanol, hexanal, 1-heptanol, 2-ethyl-1-propanol, octanol, 2-ethyl-1-hexanol, octanol, and nonanol. These organic compounds having 2 or more carbon atoms can be used as a chemical industry raw material, and among these, a mixture of organic compounds having 4 or more carbon atoms can be used as a fuel composition.

As for a raw material alcohol used in the first synthesizing method described herein, it is 2 or more kinds of alcohol, and it may be 2 kinds of alcohol, or 3 or more kinds of alcohol. Further, raw material alcohol may be a linear alcohol or branched-chain alcohol, and may be a saturated alcohol or unsaturated alcohol. Further, the number of carbon atoms is not particularly limited, but it is preferred to be an alcohol having 1-22 carbon atoms, from the point of view of easiness to obtain.

Further, it is preferred that at least 1 kind of alcohol of the raw material alcohol, is methanol or ethanol. By using methanol or ethanol for at least 1 kind of alcohol, organic compounds can be synthesized in good yield.

Particularly, according to a method described herein, allowing ethanol and linear alcohol other than ethanol to contact hydroxyapatite, it is possible to synthesize a liner alcohol having 3 or more atoms in good yield. The yield is for example, 3C-mol% or more, and preferably 5C-mol% or more. C-mol denotes the number of carbon atoms of the synthesized alcohol/the number of carbon atoms of raw material alcohol used. As for the linear alcohol other than ethanol, from the view point of easiness to obtain or cost, a saturated or unsaturated alcohol having 1-22 carbon atoms is preferred, and a saturated or unsaturated alcohol having 1-8 carbon atoms is more preferred. Specific examples include methanol, 1-propanol, 1-butanol, 1-pentanol, 1-hexanol, 1-heptanol, 1-octanol, and unsaturated alcohol thereof. Further, the amount used (mixing ratio) of ethanol and linear alcohol other than ethanol is not particularly limited, while in order to synthesize linear alcohol more efficiently, it is preferred that the mixing ratio is approximately equimolar (about 1 : 0.9-1.1) when the conversion rates of the two alcohols are almost the same. When the conversion rates of the two alcohols are different, it is preferred to mix a larger amount of alcohol with the lower conversion rate. Specifically, when using ethanol and 1-propanol, it is particularly preferred to use ethanol in an amount of about 0.9 - 1.1 (molar ratio) per 1 portion of 1-propanol.

According to a method comprising allowing methanol and alcohol having 3 or more carbon atoms to contact hydroxyapatite, it is possible to synthesize a branched-chain alcohol in good yield. As for the above alcohol having 3 or more carbon atoms, from the view point of easiness to obtain or cost, a saturated or unsaturated alcohol having 3-22 carbon atoms is preferred, and a saturated or unsaturated alcohol having 3-8 carbon atoms is more preferred. Specific examples include propanol, butanol, pentanol, hexanol, heptanol, octanol, and unsaturated alcohol thereof. Among these, linear alcohol is preferred, and specific examples include 1-propanol, 1-butanol, 1-pentanol, 1-hexanol, 1-heptanol, 1-octanol, and unsaturated alcohol thereof. Further, the amount used (mixing ratio) of methanol and alcohol having 3 or more carbon atoms is not particularly limited, while it is preferred to use 0.9 or more (molar ratio) of methanol per 1 alcohol having 3 or more carbon atoms, from the view point that a branched-chain alcohol is synthesized in good yield.

Further, as for a method for synthesizing an organic compound described herein (second synthesizing method), it is not particularly limited as long as it is a method allowing 1 kind of alcohol having 3 or more carbon atoms to contact hydroxyapatite (except those supporting metal catalysts or metal ion catalysts acting on alcohol). Examples of organic compounds synthesized by the synthesizing method described herein include, similarly as the above first synthesizing method, paraffins, olefins, dienes, trienes, alcohols, ethers, ketones, aldehydes, and esters. Among these, each organic compound having 2 or more carbon atoms can be used as chemical industry raw material. Further, a mixture of organic compounds having 4 or more carbon atoms can be used as a fuel composition.

As for the above alcohol having 3 or more carbon atoms, it may be a linear alcohol or branched-chain alcohol, and it may be a saturated alcohol or unsaturated alcohol. Further, the number of carbon atoms is not particularly limited, while from the view point of easiness to obtain or cost, a saturated or unsaturated alcohol having 3 - 22 carbon atoms is preferred, and a saturated or unsaturated alcohol having 3 - 8 carbon atoms is more preferred. Specific examples include propanol, butanol, pentanol, hexanol, heptanol, octanol, and unsaturated alcohol thereof. Among these, linear alcohol is preferred, and specific examples include 1-propanol, 1-butanol, 1-pentanol, 1-hexanol, 1-heptanol, 1-octanol, and unsaturated alcohol thereof.

Hydroxyapatite used in the synthesizing method of the present invention (or the first and second synthesizing methods described herein) is one kind of calcium phosphate, and is generally indicated by the stoichiometric composition Ca₁₀(PO₄)₆(OH)₂. However, it can form an apatite structure, showing a property of hydroxyapatite, even it is a hydroxyapatite with a non-stoichiometric composition wherein the Ca/P molar ratio does not reach 1.67. Such synthesized hydroxyapatite with a Ca/P molar ratio of approximately 1.4 - 1.8 is also encompassed within the hydroxyapatite of the present invention. Particularly, in a method for synthesizing a branched-chain alcohol of the present invention, a hydroxyapatite with a Ca/P molar ratio of 1.60 - 1.80 is preferred. The hydroxyapatite may be in any form including granule, sphere, pellet, and honeycomb.

Further, the hydroxyapatite used in the synthesizing method of the present invention does not encompass those supporting metal catalysts or metal ion catalysts acting on alcohol. Examples of metal catalyst or metal ion catalyst acting on alcohol include metals or metal ions described in Japanese Laid-Open Patent Application No. 5-305238.

The hydroxyapatite used in the synthesizing method of the present invention may support in advance 1 kind of raw material alcohol such as methanol. In other words, before conducting the synthesizing reaction of organic compounds, it is possible to allow hydroxyapatite to react with 1 kind of raw material alcohol, and to use alcohol-supported hydroxyapatite, wherein hydroxyapatite is supporting alcohol. The absorption peak derived from alcohol of the alcohol-supported hydroxyapatite can be observed by infrared spectroscopy. By using the alcohol-supported hydroxyapatite, distribution of reaction products can be controlled. In other words, many products derived from supported alcohol can be synthesized.

In the synthesizing method described herein, at least one compound selected from the group consisting of metal oxidative product, zeolite, silica light, clay mineral of the family of kaolin, clay mineral of the family of pyrophyllite, clay mineral of the family of smectite, hydrotalcite, sepiolite, calcium silicate, calcium fluoride, calcium sulfate, apatite fluoride, magnesium hydroxide, chitin, lithium phosphate, aluminum phosphate, and magnesium phosphate, can be mixed to the above hydroxyapatite for controlling the reaction. 2 or more of these compounds can be used in combination.

In the present invention, when synthesizing a branched-chain alcohol useful as a chemical industry raw material, in order to increase the selectivity of desired organic compounds, the size, surface area, reaction conditions (contact time, reaction temperature, pressure, etc.) of granules used can be appropriately selected.

As for a reaction form in the present invention, it may be a batch method or a sequential method, while a continuous method is preferred from the view point of industrial economic efficiency. Further, a reactor in any form including a fixed bed, a moving bed, a fluidized bed or a slurry bed can be used. Moreover, it may be a liquid phase reaction or a gas phase reaction, and the reaction may be conducted at normal pressure, under pressure, or reduced pressure. In case of a gas phase reaction, a mixed alcohol gas alone may be in contact with hydroxyapatite, or it may be in contact with hydroxyapatite together with an inert carrier gas such as nitrogen or helium. By allowing to contact together with a carrier gas, unnecessary retention of raw material and products may be suppressed, and the reaction may be conducted more efficiently. At that time, in order to maintain the catalyst activity, reactive gas such as hydrogen, hydrocarbon, and water may be accompanied in the carrier gas. Further, in order to prevent that carbons are precipitated on the surface of hydroxyapatite, which may decrease the alcohol conversion rate and change the nature of reactions, it is preferred that a regeneration treatment wherein the hydroxyapatite is heated under oxygen atmosphere, is periodically conducted. In other words , it is preferred that a catalyst regeneration apparatus that is capable of conducting a regeneration treatment as above-mentioned is provided on the reactor.

It is not possible to determine categorically the contact time of alcohol and hydroxyapatite as it affects also the reaction temperature. Generally, in case of a gas phase reaction by a continuous method, the contact time is about 0.1 - 20 sec, and preferably about 0.4 - 5 sec. Further, the reaction temperature is generally 100 - 700°C, and preferably 200 - 600°C. Particularly, when synthesizing in good yield a linear alcohol by using ethanol and linear alcohol other than ethanol, the reaction temperature is preferably 250 - 450°C, and more preferably 300 - 450°C. Further, when synthesizing a branched-chain alcohol in good yield by using methanol and alcohol having 3 or more carbon atoms, the reaction temperature is preferably 250 - 500°C, and more preferably 300 - 450°C.

When conducting a gas phase reaction with 2 or more kinds of alcohol, it is preferred to vaporize the alcohol-mixed solution, and it is preferred to vaporize rapidly, without allowing the reaction of 2 or more kinds of alcohol to be conducted. Therefore, as for the vaporizing temperature, a temperature that is higher than the boiling point of the alcohol having the higher boiling point, and at which the alcohol with the lower boiling point does not react is preferred. Specifically, the preferred temperature is, in case of methanol and ethanol, 150 - 200°C, and in case of ethanol and 1-octanol, 200 - 250°C.

As the boiling points of 2 or more kinds of alcohol are different, 1 kind of alcohol having been vaporized may be firstly introduced to form a complex catalyst supporting alcohol, and then the other alcohol in form or liquid or gas may be introduced to start the reaction (liquid phase reaction, gas phase reaction). When using methanol or ethanol, it is preferred to firstly introduce methanol or ethanol having a low boiling point, and to form a complex catalyst supporting methanol or ethanol. Generally, the order of alcohol to be introduced may be determined according to the boiling point as in the above, while when using ethanol, it is preferred to introduce ethanol in the first order.

A mixture of organic compounds thus obtained, may be used as a fuel composition etc. directly in form of mixture. Alternatively, a desired organic compound may be separated or purified according to a conventional separation or purification method, for example by rectification, microporous membrane separation, extraction, or adsorption.

Further, as for a method for synthesizing an organic compound described herein (third synthesizing method), it is not particularly limited as long as it is a method allowing 2 or more kinds of alcohol to contact hydrotalcite. Organic compounds synthesized by the synthesizing method described herein, are similar to the above case using hydroxyapatite, and various organic compounds may be synthesized in good yield. Particularly, when ethanol and linear alcohol other than ethanol is used, a linear alcohol having 3 or more carbon atoms can be synthesized in good yield.

Hydrotalcite used as described herein is a clay mineral having a composition of Mg₆Al₂ (OH) ₁₆CO₃·4H₂O. Similarly to the above-mentioned hydroxyapatite, it may support alcohol beforehand.

Raw material alcohol or synthesizing method is similar to that of the above first or second synthesizing method, and thus the detailed explanation is abbreviated.

The present invention will be explained in more detail in the following by referring to the Examples.

### Examples

### [First Synthesizing Method]

### [Example 1-1]

### (Catalyst)

As for a catalyst used in the Example, a hydroxyapatite prepared by a common method was used. In the Table, "HAP1" denotes a hydroxyapatite which Ca/P molar ratio is 1.66, "HAP2" denotes a hydroxyapatite which Ca/P molar ratio is 1.64, and "HAP3" denotes a hydroxyapatite which Ca/P molar ratio is 1.61. As for a catalyst used in the comparative example, as Mg(OH)₂, MgO reagent (Wako Pure Chemicals) boiled and hydrated in distilled water (see Ueda, W.; Kuwabara, T.; Ohshida, T.; Morikawa, Y. A Low-pressure Guerbet Reaction over Magnesium Oxide Catalyst. J. Chem. Soc., Chem. Commun., 1990, 1558-1559), as for ZrO2, a reference catalyst of Catalyst (JRC-ZRO-5), and as for others, reagents from Wako Pure Chemicals were used, respectively.

### (Evaluation of catalyst property)

A fixed bed gas flow catalytic reactor (Ohkura Riken) was used as a reactor. 0.2 - 4 cc of hydroxyapatite was filled in a silica reaction tube with a diameter of 5 mm. As a pretreatment, thermal dehydration treatment was conducted for 30 min under a carrier gas atmosphere (1% Ar/He base; flow 112 ml/min) at 500°C. Following the pretreatment, mixed alcohol gas diluted with helium (alcohol concentration 20 vol%) was introduced so that GHSV becomes 500 - 10000 (1/h) to allow reaction at normal pressure. For the reaction temperature, a sampling was conducted every 50°C from 100-500°C. A gas chromatography mass spectrometer (GC-MS) was used for the identification of the components of the reaction gas, and a gas chromatography (GC) (detector: FID) was used for the measurement of the alcohol conversion rate and the selectivity of the synthetic gas, to quantify the amount of each component from the peak surface value of each component. For each test, the yield of organic compounds having 2 or more carbon atoms (C2+), organic compounds having 4 or more carbon atoms (C4+), alcohol (linear and branched-chain), and linear alcohol were measured. The results are shown in Tables 1 - 4 . In the Tables, "n-C" denotes normal alcohol, "b-C" denotes branched chain alcohol, and "C=" denotes unsaturated alcohol.

### (Organic compounds having 2 or more carbon atoms) (C2+))

Yield of organic compounds of C2 or more in various combinations of raw material alcohols is shown in Table 1. Test examples 2 and 3 are examples according to the invention, the other test examples are reference examples.

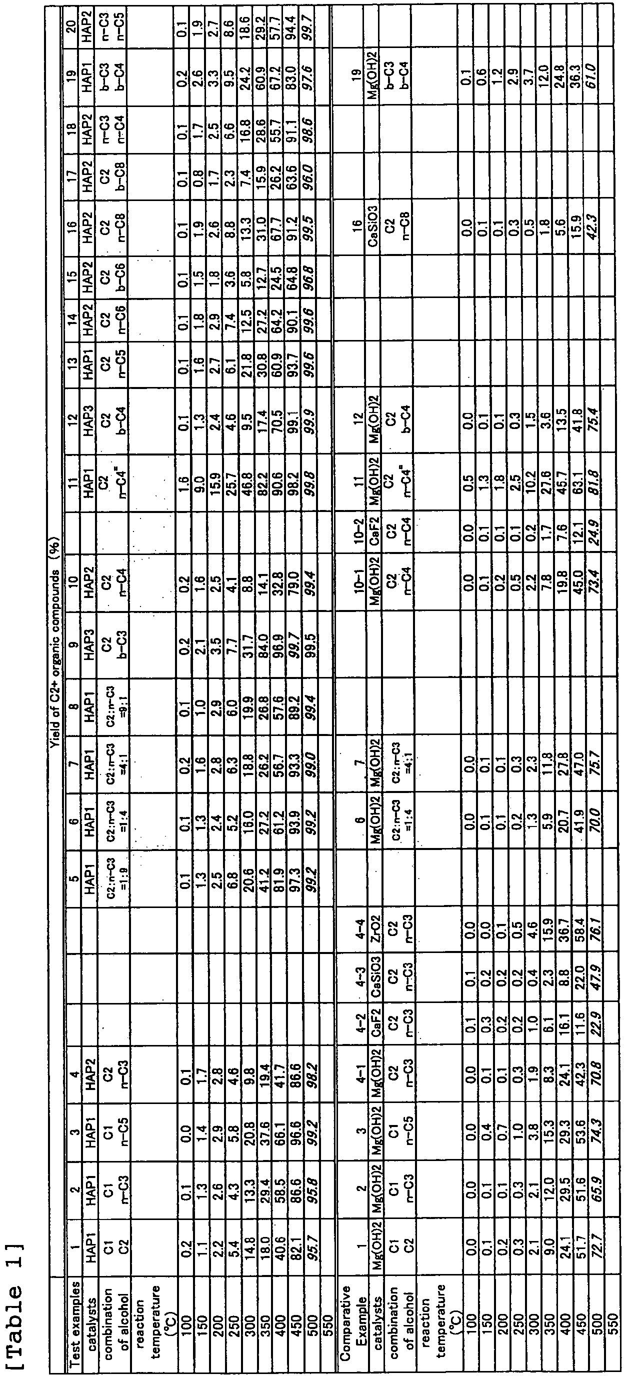

### (Organic compounds having 4 or more carbon atoms) (C4+))

Yield of organic compounds of C4 or more in various combinations of raw material alcohols is shown in Table 2.

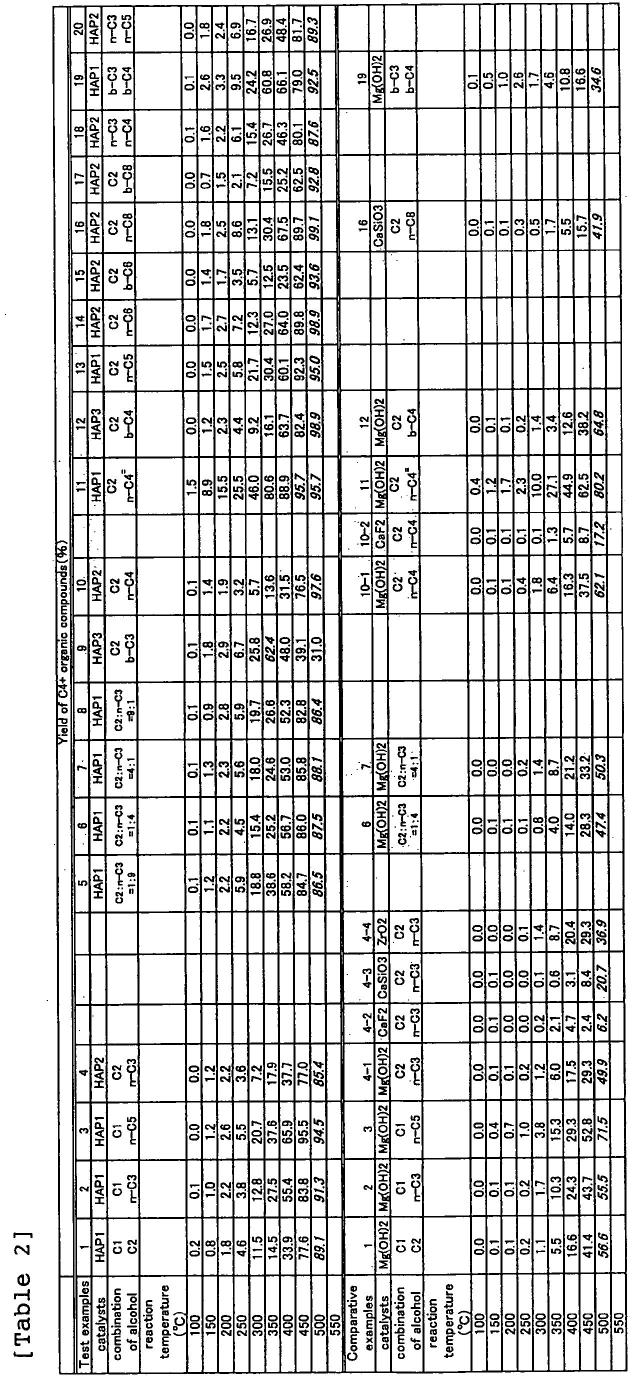

### (alcohols (linear and branched-chain))

Yield of alcohols (linear and branched-chain) in various combinations of raw material alcohols is shown in Table 3.

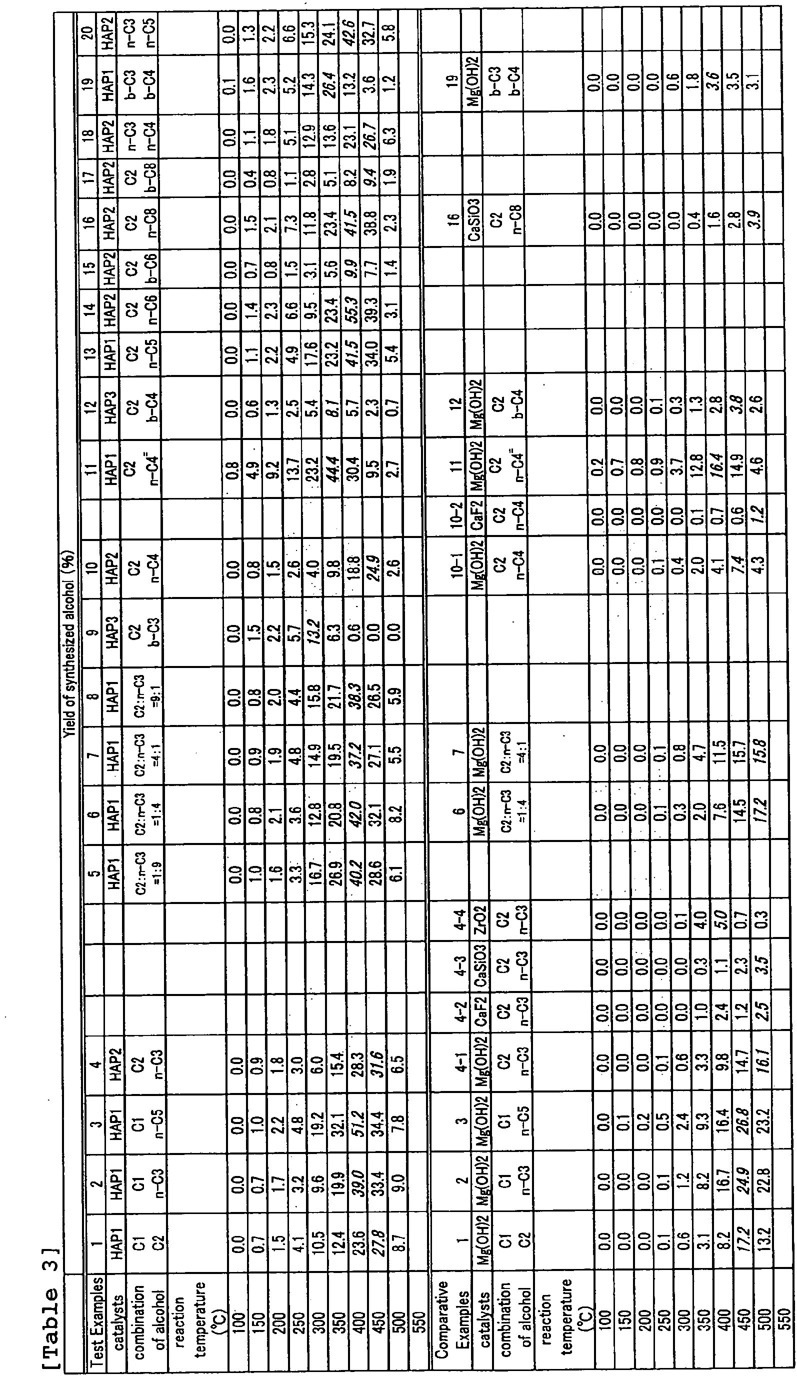

### (Linear alcohol)

Yield of linear alcohol in various combinations of raw material alcohols is shown in Table 4. Yield of linear alcohol in Table 4 shows the yield of linear alcohol synthesized directly from 2 kinds of raw material alcohols. For example, when methanol (C1) and ethanol (C2) are used as raw materials, yield of 1-propanol is shown. When ethanol (C2) and 1-propanol (C3) are used as raw materials, yield of 1-pentanol (C5) is shown.

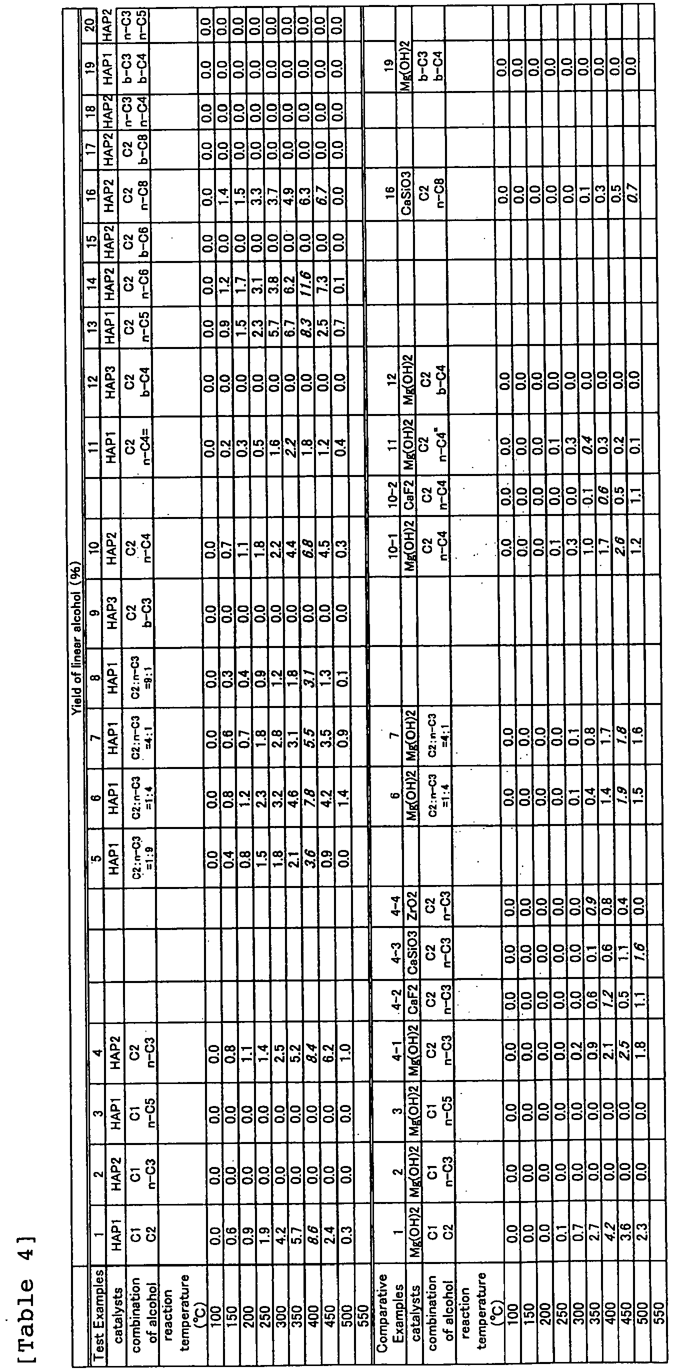

As it is clear from Tables 1 and 2, according to the synthesizing method described herein, organic compounds useful as a chemical industry raw material can be synthesized in good yield. Further, as it is clear from Tables 3 and 4, when ethanol and linear alcohol other than ethanol are used, liner alcohol is synthesized in good yield. When methanol and alcohol having 3 or more carbon atoms are used, branched-chain alcohol is synthesized in good yield.

### [Example 1-2, reference example]

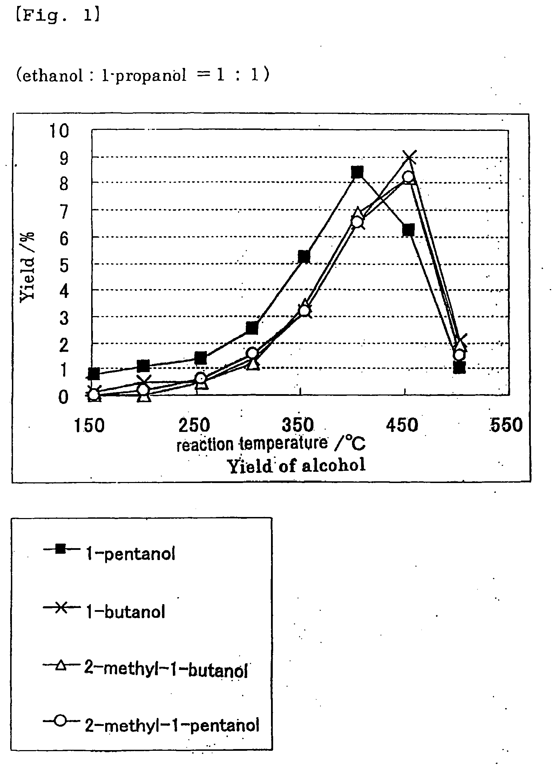

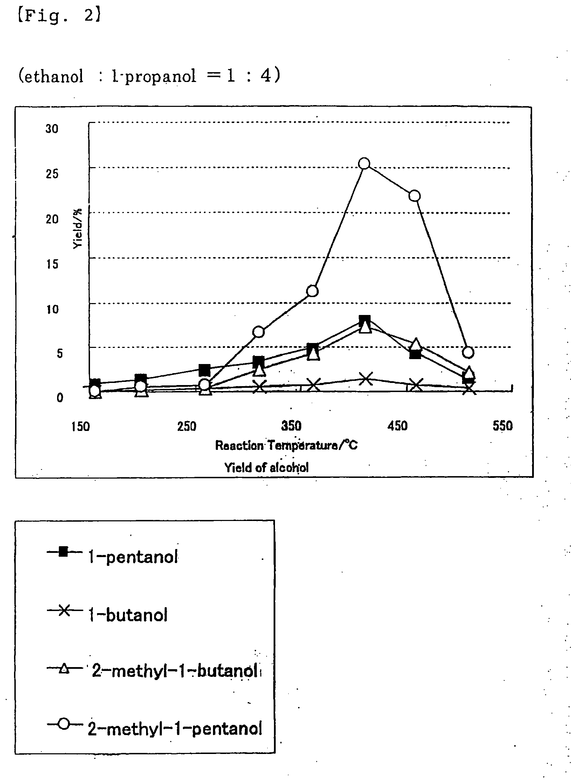

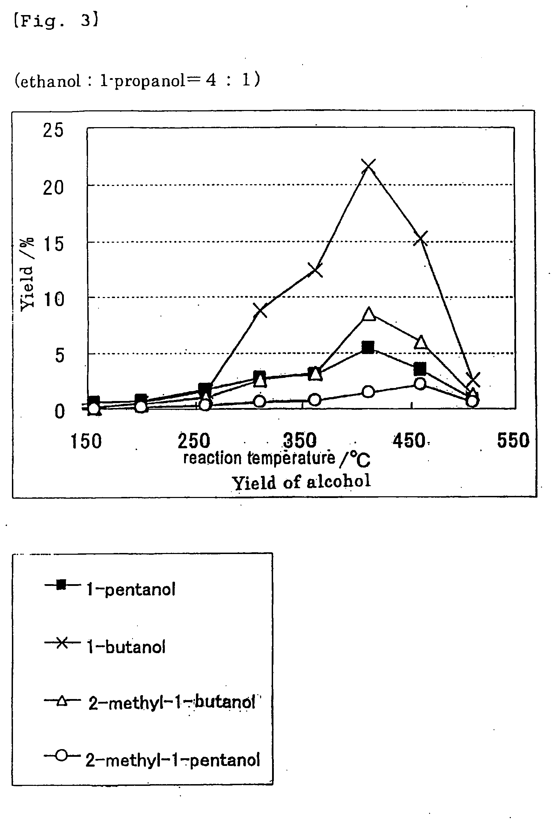

Ethanol and 1-propanol were used as raw material alcohols, and by changing the mixing ratio, reaction was conducted similarly as Example 1 - 1. Yield of alcohol against the reaction temperature is shown in Figs. 1 - 3.

As it is clear from Figs. 1-3, kinds of alcohol produced vary depending on the mixing ratio (molar ratio). When the mixing ratio is 1:1, 1-pentanol which is a linear alcohol is synthesized the most. Therefore, when synthesizing linear alcohol having odd number of carbon atoms, such as 1-pentanol, when the converting ratio of raw material alcohol is almost equal, it is preferred that the mixing ratio is about 1:1.

### [Second synthesizing method]

### [Example 2, reference example]

### (Catalysts)

The same catalysts as Example 1 were used.

### (Estimation of Catalyst properties)

Reaction was conducted similarly as Example 1, except for using 1 kind of alcohol having 3 or more carbon atoms as raw material alcohol. For each test, yield of organic compounds of C2 or more, organic compounds of C4 or more, alcohol (linear and branched-chain), and linear alcohol were measured. The results are shown in Tables 5-7. Linear alcohol was not produced in any of the Examples or Comparative Examples.

### (Organic compounds having 2 or more carbon atoms (C2+))

Yield of organic compounds of C2 or more for each raw material alcohol is shown in Table 5.

**[Table 5]**

| | Yield of C2+ organic compounds (%) | | | | |
|---|---|---|---|---|---|
| Test Examples | 21 | 22 | 23 | 24 | 25 |
| catalysts | HAP2 | HAP2 | HAP2 | HAP1 | HAP2 |
| combination of alcohol | n-C3 | b-C3 | n-C4 | n-C4= | b-C4 |
| reaction temperature (°C) | | | | | |
| 100 | 0.1 | 0.1 | 0.2 | 3.8 | 0.1 |
| 150 | 1.5 | 1.8 | 1.5 | 5.9 | 1.6 |
| 200 | 2.7 | 3.1 | 2.3 | 10.5 | 2.8 |
| 250 | 4.2 | 9.9 | 3.8 | 12.8 | 5.6 |
| 300 | 22.5 | 33.4 | 8.0 | 23.7 | 17.3 |
| 350 | 43.2 | 94.0 | 14.5 | 45.8 | 66.7 |
| 400 | 84.6 | *99.9* | 35.8 | 99.6 | 96.8 |
| 450 | *99.7* | 99.7 | *88.1* | *99.9* | 99.2 |
| 500 | 99.5 | 99.5 | 96.8 | *99.9* | *99.8* |
| 550 | | | | | |

| Comparative Examples | 21 | 22 | 23 | 24 | 25 |
|---|---|---|---|---|---|
| catalysts | Mp(OH)2 | CaF2 | ZrO2 | Mg(OH)2 | CaSiO4 |
| combination of alcohol | n-C3 | b-C3 | n-C4 | n-C4= | b-C4 |
| reaction temperature (°C) | | | | | |
| 100 | 0.0 | 0.0 | 0.0 | 1.3 | 0.1 |
| 150 | 0.1 | 0.2 | 0.1 | 2.3 | 0.2 |
| 200 | 0.2 | 0.2 | 0.1 | 2.7 | 0.2 |
| 250 | 0.3 | 0.3 | 0.2 | 3.5 | 0.2 |
| 300 | 2.1 | 1.5 | 5.6 | 4.2 | 0.8 |
| 350 | 8.5 | 6.8 | 9.3 | 6.7 | 1.8 |
| 400 | 24.9 | 12.0 | 25.2 | 26.6 | 5.5 |
| 450 | 43.6 | 17.1 | 51.6 | 48.5 | 19.8 |
| 500 | *72.1* | *23.7* | *67.7* | *76.1* | *55.7* |
| 550 | | | | | |

### (Organic compounds having 4 or more carbon atoms (C4+))

Yield of organic compounds of C4 or more for each raw material alcohol is shown in Table 6.

**[Table 6]**

| | Yield of C4+ organic compounds (%) | | | | |
|---|---|---|---|---|---|
| Test Examples | 21 | 22 | 23 | 24 | 25 |
| catalysts | HAP2 | HAP2 | HAP2 | HAP1 | HAP2 |
| combination of alcohol | n-C3 | b-C3 | n-C4 | n-C4= | b-C4 |
| reaction temperature (°C) | | | | | |
| 100 | 0.1 | 0.1 | 0.2 | 3.6 | 0.1 |
| 150 | 1.4 | 1.2 | 1.4 | 5.8 | 1.6 |
| 200 | 2.6 | 2.3 | 2.2 | 10.3 | 2.7 |
| 250 | 4.0 | 6.2 | 3.7 | 12.5 | 5.5 |
| 300 | 21.8 | 23.6 | 7.9 | 23.3 | 17.1 |
| 350 | 38.8 | *63.7* | 14.4 | 45.2 | 66.6 |
| 400 | *65.2* | 37.9 | 35.7 | 98.3 | 96.7 |
| 450 | 61.2 | 22.3 | 87.4 | *98.5* | 99.0 |
| 500 | 55.5 | 17.2 | *94.6* | 97.7 | *99.2* |
| | | | | | |

| Comparative Examples | 21 | 22 | 23 | 24 | 25 |
|---|---|---|---|---|---|
| catalysts | Mp(OH)2 | CaF2 | ZrO2 | Mg(OH)2 | CaSiO4 |
| combination of alcohol | n-C3 | b-C3 | n-C4 | n-C4= | b-C4 |
| reaction temperature (°C) | | | | | |
| 100 | 0.0 | 0.0 | 0.0 | 1.2 | 0.0 |
| 150 | 0.1 | 0.1 | 0.1 | 2.2 | 0.1 |
| 200 | 0.1 | 0.1 | 0.1 | 2.5 | 0.1 |
| 250 | 0.2 | 0.2 | 0:2 | 3.1 | 0.2 |
| 300 | 1.1 | 0.7 | 5.5 | 3.6 | 0.7 |
| 350 | 6.3 | 4.2 | 9.1 | 6.2 | 1.6 |
| 400 | 17.4 | 5.4 | 24:6 | 25.3 | 5.3 |
| 450 | 27.6 | 8.6 | 57.7 | 46.1 | 18.3 |
| 500 | *43.7* | *11.4* | *64.2* | *73.8* | *52.6* |
| 550 | | | | | |

### (Alcohol (linear and branched-chain)

Yield of alcohol (linear and branched-chain) for each raw material alcohol is shown in Table 7.

**[Table 7]**

| | Yield of total synthesized alcohol (%) | | | | |
|---|---|---|---|---|---|
| Test Examples | 21 | 22 | 23 | 24 | 25 |
| catalysts | HAP2 | HAP2 | HAP2 | HAP1 | HAP2 |
| combination of alcohol | n-C3 | b-C3 | n-C4 | n-C4= | b-C4 |
| reaction temperature (°C) | | | | | |
| 100 | 0.0 | 0.0 | 0.0 | 1.1 | 0.0 |
| 150 | 1.2 | 1.0 | 1.2 | 2.3 | 0.8 |
| 200 | 2.2 | 1.7 | 1.8 | 2.8 | 1.2 |
| 250 | 3.5 | 3.2 | 2.7 | 3.2 | 2.3 |
| 300 | 18.4 | *5.6* | 3.8 | 4.6 | 3.5 |
| 350 | *28.2* | 2.5 | 5.3 | 15.6 | *6.0* |
| 400 | 14.9 | 0.8 | 8.8 | *27.9* | 2.0 |
| 450 | 2.4 | 0.2 | 17.4 | 8.6 | 0.7 |
| 500 | 1.8 | 0.0 | *18.0* | 3.0 | 0.0 |
| 550 | | | | | |

| Comparative Examples | 21 | 22 | 23 | 24 | 25 |
|---|---|---|---|---|---|
| catalysts | M_{g}(OH)2 | CaF2 | ZrO2 | Mg(OH)2 | CaSiO4 |
| combination of alcohol | n-C3 | b-C3 | n-C4 | n-C4= | b-C4 |
| reaction temperature (°C) | | | | | |
| 100 | 0.0 | 0.0 | 0.0 | 0.2 | 0.0 |
| 150 | 0.0 | 0.0 | 0.0 | 0.3 | 0.0 |
| 200 | 0.1 | 0.0 | 0.1 | 0.2 | 0.0 |
| 250 | 0.2 | 0.0 | 0.2 | 0.2 | 0.0 |
| 300 | 0.7 | 0.2 | 0.7 | 0.3 | 0.1 |
| 350 | 3.2 | 0.6 | 0.9 | 1.5 | 0.2 |
| 400 | 7.7 | 0.7 | 1.7 | 2.6 | 0.3 |
| 450 | 11.4 | 1.2 | 3.9 | *4.4* | 0.5 |
| 500 | *14.6* | *1.5* | *5.5* | 1.3 | *0.7* |
| 550 | | | | | |

As it is clear from Tables 5 - 7, according to the synthesizing method described herein, organic compounds useful as a chemical industry raw material can be synthesized in good yield.

### [Third synthesizing method

### [Example 3, reference example]

### (Catalyst)

Hydrotalcite (Wako Pure Chemicals) was used as a catalyst.

### (Estimation of Catalyst properties)

Reaction was conducted similarly as Example 1, except for using hydrotalcite instead of hydroxyapatite. For each test, yield of organic compounds of C2 or more (C2+), organic compounds of C4 or more (C4+), alcohol (linear and branched-chain), and linear alcohol were measured. The results are shown in Table 8.

**[Table 8]**

| Test Examples | 26 | 26 | 26 | 26 |
|---|---|---|---|---|
| yield (%) | C2+ | C4+ | total alcohol | linear alcohol |
| catalysts | hydrotalcite | hydrotalcite | hydrotalcite | hydrotalcite |
| combination of alcohol | C2+n-C3 | C2+n-C3 | C2+n-C3 | C2+n-C3 |
| reaction temperature (°C) | | | | |
| 100 | 0.0 | 0.0 | 0.0 | 0.0 |
| 150 | 0.5 | 0.4 | 0.2 | 0.1 |
| 200 | 1.1 | 0.7 | 0.4 | 0.3 |
| 250 | 2.2 | 1.9 | 1.0 | 0.6 |
| 300 | 4.9 | 4.2 | 1.9 | 1.0 |
| 350 | 12.8 | 10.3 | 4.2 | 2.3 |
| 400 | 35.8 | 26.1 | 9.4 | *4.1* |
| 450 | 71.5 | 44.5 | *13.6* | 3.6 |
| 500 | *92.4* | *55.6* | 6.6 | 0.9 |
| 550 | | | | |

| Test Examples | 27 | 27 | 27 | 27 |
|---|---|---|---|---|
| yield (%) | C2+ | C4+ | total alcohol | linear alcohol |
| catalysts | hydrotalcite | hydrotalcite | hydrotalcite | hydrotalcite |
| combination of alcohol | C2+n-C4 | C2+n-C4 | C2+n-C4 | C2+n-C4 |
| reaction temperature (°C) | | | | |
| 100 | 0.0 | 0.0 | 0.0 | 0.0 |
| 150 | 1.4 | 1.2 | 0.8 | 0.5 |
| 200 | 2.0 | 1.7 | 1.1 | 0.6 |
| 250 | 3.4 | 3.1 | 1.3 | 0.8 |
| 300 | 5.1 | 4.8 | 1.6 | 1.0 |
| 350 | 14.7 | 13.6 | 3.1 | 2.2 |
| 400 | 31.6 | 27.5 | 5.2 | *3.4* |
| 450 | 69.6 | 58.5 | *6.4* | 2.6 |
| 500 | *88.7* | *76.8* | 2.7 | 0.8 |
| 550 | | | | |

As it is clear from Table 8, according to the synthesizing method described herein, organic compounds useful as a chemical industry raw material can be synthesized in good yield.

### [Reference Example]

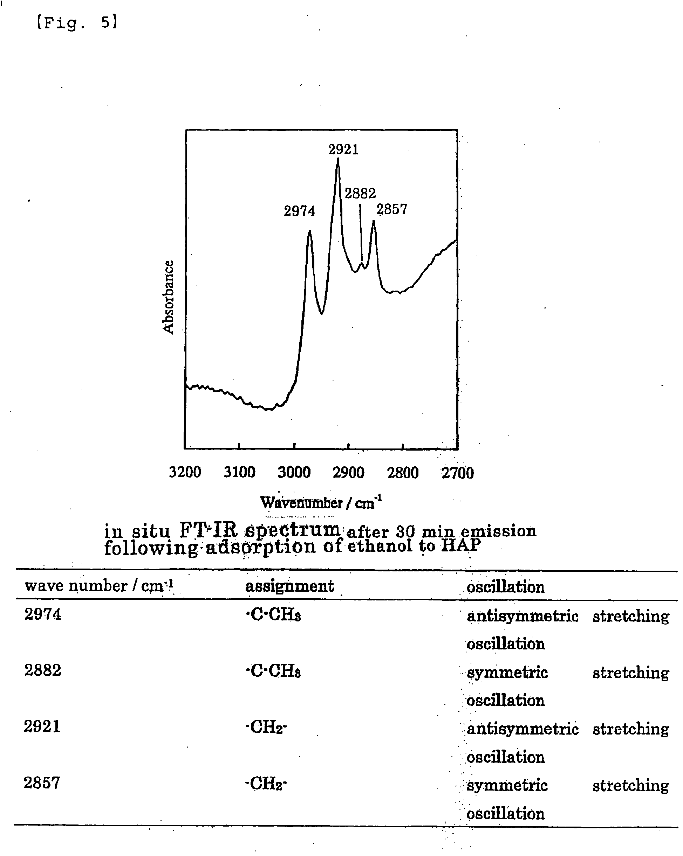

Hydroxyapatite (HAP1) was exposed to about 7 vol% ethanol/He mixed gas for 1 hour in a reactor, and then emission was conducted. The inner state of the reactor after 1 hour of exposure to the mixed gas and the following 30 min of emission was measured by in situ FT-IR with a diffuse reflection method. The results are shown in Figs 4 and 5. In Fig. 4, the upper spectrum shows the state after 1 hour exposure to the mixed gas, and the lower spectrum shows the state after 30 min emission. As it is clear from Figs. 4 and 5, it can be observed that ethanol is absorbed and supported by hydroxyapatite.

### Industrial Applicability

According to the method for synthesizing alcohol described herein, various organic compounds can be manufactured from 2 or more kinds of alcohols or from 1 kind of alcohol having 3 or more carbon atoms . Particularly, when using 2 or more kinds of alcohols, linear alcohol or branched-chain alcohol can be synthesized in good yield.

## Claims

1. A method for synthesizing 1 or more kinds of branched-chain alcohol comprising allowing methanol and alcohol having 3 or more carbon atoms to contact hydroxyapatite at normal pressure and at 200 - 600°C, wherein the hydroxyapatite is not one supporting metal catalysts or metal ion catalysts acting on alcohol.

2. The method according to claim 1, wherein the alcohol having 3 or more carbon atoms is a linear alcohol.

3. The method according to claim 2, wherein the linear alcohol is 1-propanol, 1-butanol, 1-pentanol, 1-hexanol, 1-heptanol, 1-octanol, or unsaturated alcohols thereof.

## Patentansprüche

1. Verfahren zur Synthese von 1 oder mehreren Arten von verzweigten Alkoholen, bei dem man Methanol und Alkohol mit 3 oder mehr Kohlenstoffatomen bei Normaldruck und bei 200 - 600 °C mit Hydroxylapatit in Kontakt bringt, wobei es sich bei dem Hydroxylapatit nicht um einen handelt, der Metallkatalysatoren oder Metallionenkatalysatoren, die auf Alkohole wirken, trägert.

2. Verfahren nach Anspruch 1, wobei es sich bei dem Alkohol mit 3 oder mehr Kohlenstoffatomen um einen linearen Alkohol handelt.

3. Verfahren nach Anspruch 2, wobei es sich bei dem linearen Alkohol um 1-Propanol, 1-Butanol, 1-Pentanol, 1-Hexanol, 1-Heptanol, 1-Octanol oder ungesättigte Alkohole davon handelt.

## Revendications

1. Procédé pour la synthèse de 1 ou plusieurs sortes d'alcool à chaîne ramifiée comprenant le fait de laisser du méthanol et un alcool possédant 3 atomes de carbone ou plus entrer en contact avec une hydroxyapatite à pression normale et à une température de 200 à 600 °C, l'hydroxyapatite n'en étant pas une qui supporte des catalyseurs métalliques ou des catalyseurs à ion métallique agissant sur un alcool.

2. Procédé selon la revendication 1, l'alcool possédant 3 atomes de carbone ou plus étant un alcool linéaire.

3. Procédé selon la revendication 2, l'alcool linéaire étant le 1-propanol, le 1-butanol, le 1-pentanol, le 1-hexanol, le 1-heptanol, le 1-octanol, ou des alcools insaturés correspondants.
